Europäisches Patentamt

European Patent Office

Office européen des brevets

(11) Publication number: **0 502 994 B1**

# EUROPEAN PATENT SPECIFICATION

(45) Date of publication of patent specification: **06.09.95** (51) Int. Cl.⁶: **C12Q 1/70,** //C12N15/37

(21) Application number: **91901374.8**

(22) Date of filing: **03.12.90**

(86) International application number:
**PCT/US90/07057**

(87) International publication number:
**WO 91/08312 (13.06.91 91/13)**

Divisional application 95200344.0 filed on
03/12/90.

(54) **DETECTION OF HPV TRANSCRIPTS.**

(30) Priority: **01.12.89 US 444526**

(43) Date of publication of application:
**16.09.92 Bulletin 92/38**

(45) Publication of the grant of the patent:
**06.09.95 Bulletin 95/36**

(84) Designated Contracting States:
**DE FR GB IT**

(56) References cited:
**EP-A- 0 192 001      EP-A- 0 256 321
EP-A- 0 301 968      EP-A- 0 373 352
EP-A- 0 402 132      WO-A-87/05630
WO-A-88/06634        WO-A-89/09940
WO-A-90/02821        US-A- 4 849 332**

**JOURNAL OF EXPERIMENTAL MEDICINE, vol.
167, January 1988; D.K. SHIBATA et al., pp.
225-230/**

(73) Proprietor: **AMOCO CORPORATION
200 East Randolph Drive
P.O. Box 87703
Chicago
Illinois 60680-0703 (US)**

(72) Inventor: **HENDRICKS, David, A.
2034 Del Norte Street
Berkeley, CA 94707-2403 (US)**
Inventor: **LANE, David, J.
9 Oriole Drive
Milton, MA 01757 (US)**
Inventor: **RIGBY, Susan
23 Deering Avenue
Lexington, MA 02173 (US)**
Inventor: **PARODOS, Kyriaki
30 Royal Crest Drive,
Apt. 9
Marlborough, MA 01752 (US)**

JOURNAL OF MOLECULAR BIOLOGY, vol. 193, 1987; S.T. COLE et al., pp. 599-608/

BULLETIN OF CANCER, vol. 74, 1987; M. IONESCU et al., pp. 397-406/

(74) Representative: **Holdcroft, James Gerald, Dr. et al**
**Graham Watt & Co.,**
**Riverhead**
**Sevenoaks, Kent TN13 2BN (GB)**

## Description

Background

Epidemiologic data are accumulating which show that the human papillomavirus (HPV) is strongly associated, as a factor or co-factor, with cervical cytologic abnormalities, such as cervical intraepithelial neoplasias (CIN) and carcinoma in situ (CIS). Syrjanen, K. J., In: Papillomaviruses and Human Disease, K. Syrjanen, L. Gissman, and L.G. Koss (Eds.), Springer-Verlag, pp 467-503, (1987), In general, these dysplasias are found in the transition zone of the cervix and are graded from mild to severe (I to III), based on the extent to which neoplastic cells extend from the basal layer to the epithelial surface. Complete replacement of the epithelium by neoplastic cells is termed carcinoma in situ (CIS).

At least 60 types of HPV, isolated from various parts of the human body, have been documented, and more than 20 of these types have been shown to be associated with the genital mucosa. E. M. de Villiers, J. Virol., 63(11):4898-4903 (1989). Although present information strongly supports the close association between HPV and cervical neoplasia, and shows that many individuals clearly have HPV DNA (i.e., infections), transiently and/or latently, it also makes it clear that women in whom HPV is present in cervical cells do not always progress to more serious dysplasia. Syrjanen et al. in Cancer Cells, Vol. 5, Cold Spring Harbor, pp. 281-288 (1987); deBrux et al. , Bull. Cancer (Paris), 70:410-422 (1983); Mitchell et al., The Lancet, 1:573-575 (1986). Thus, simple detection of HPV DNA in cervical specimens lacks discriminatory predictive value for identification of women it risk for serious disease. A method for assessing HPV activity which can be used to predict progression to serious CIN or CIS is needed.

WO-A-89/09940, EP-A-0301968, EP-A-0256321, WO-A-87/05630, EP-A-0192001 and J. Exp. Med. 167 - (1988) 225-230 disclose probes containing certain nucleic acid sequences of particular genes of particular HPV types, but do not disclose the determination of HPV E6/E7 transcriptional activity and its use in determining the prognosis of disease progression.

Summary of the Invention

The present invention relates to a method of determining the prognosis of individuals infected with HPV comprising the steps of; (1) measuring the level of HPV activity by detecting transcripts of all or a portion of the E6 and/or E7 HPV genes (and/or spliced transcripts thereof) in a sample e.g. a cervical sample, and (2) comparing the measurements of HPV activity with a previously established relationship between activity and risk of progression to serious cervical dysplasia or carcinoma.

The method of the invention is based on the association between HPV transcriptional activity and the risk for development of serious cervical dysplasia or carcinoma. For example, patterns and/or levels of transcription of at least a portion of the E6 and/or E7 genes and spliced downstream sequences of selected high-oncogenic HPV types can be used to predict the progression of HPV infection to serious CIN or CIS. Conversely, patterns and/or levels of transcription of the E6 or E7 genes of low-oncogenic HPV types can be used as an indication of reduced risk of serious CIN or CIS. Thus, the detection of E6 and/or E7 transcripts of selected HPV types in cervical samples provides a more valuable diagnostic aid than mere detection of HPV DNA.

Certain patterns of HPV transcription can provide a more reliable indicator of risk for progression to serious dysplasia than does detection of total HPV DNA. Low levels of HPV DNA are quite prevalent in women and, thus, the presence of HPV DNA is not a sensitive measure of neoplastic risk. The detection and quantitation of transcripts of genes associated with high-oncogenic or low-oncogenic types of HPV, and/or spliced mRNAs containing these sequences provide a sensitive, accurate, reliable prognosticator of serious dysplasia or cancer.

Brief Description of the Figures

Figure 1 is a schematic representation of the method of the present invention.

Figure 2 is a schematic illustration of the hybridization complex of mRNA targets, d(A)-tailed oligonucleotide capture probes, and radiolabeled detector probes for detection of E6/E7-containing transcripts of HPV.

Figure 3 shows the nucleotide sequences of thirty-three type-specific oligonucleotide probes, which range in length from 32 to 42 nucleotides.

Figure 4 shows nucleotide sequences and target sites of probes for type-specific detection of spliced E6 transcripts (E6*)of HPV 16, HPV 18, HPV 31 and HPV 38. Predicted base pairs between spliced E6* transcripts and specific oligonucleotide probes which span the upstream and downstream exons are shown.

Figure 5 is a schematic illustration of capture and detector probes on E6/E7 transcripts of HPV 16, showing the specific arrangement of probes on mRNAs of HPV 16. Splice donor (do) and acceptor (ac) sites in the E6 ORF are indicated.

Figure 6 shows nucleotide sequences of possible base pairs between HPV 16 capture probes 16-3, 16-4, and 16-5 and aligned sequences in the E7 ORF's of HPV 6, HPV 11, HPV 18, HPV 31, HPV 33 and HPV 35. Underlined bases are predicted to form base pairs with the oligonucleotide probe.

Figure 7 is a schematic illustration of the steps for cloning HPV 16 DNA into lambda 47.1 and construction of recombinant molecules for synthesis of detector probes and positive target transcripts. The strategy for engineering a construct for production of the HPV 16 riboprobe is shown on the left side of the figure and the construction of a recombinant molecule for synthesis in vitro of mRNA molecules which contain the E6 and E7 ORF's (i.e., positive target transcripts) is shown on the right side of the figure.

Figure 8 shows the relationship between the concentration of positive target and signal in an HPV 16 assay. Figure 8A shows typical results from the HPV 16 assay using a $^{32}$P-labeled detector probe with a specific activity of $5 \times 10^8$ cpm/$\mu$g. Figure 8B is a graph showing the linear relationship between the concentration of positive target and the amount of signal.

Detailed Description of the Invention

The present invention is based on an understanding of the close association between HPV and cervical carcinoma; the fact that although many individuals have HPV DNA in cervical cells, they do not progress to more serious dysplasia; and a determination that simple detection of HPV DNA in cervical specimens appears to lack discriminatory predictive value for identification of individuals at risk for progression to serious disease.

In contrast to commonly used techniques for detection of HPV DNA, the present method allows sensitive and quantitative detection of specific HPV mRNA or DNA. The present method makes it possible to assess active transcription of the HPV genome and to determine the associations between HPV transcription patterns and risk for development of cytopathology and/or progresson to more serious disease (i.e., higher grade CIN or CIS) in HPV-infected individuals. The following is a brief summary of the assessment of the role of HPV in cervical abnormalities, which establishes the need, and therefore the utility of the present method, a detailed description of the method, a description of HPV-specific nucleic acid probes useful in the method, and discussion of an application of the method in a clinical context for diagnostic and/or therapy-related purposes.

The Role of HPV in Cervical Abnormalities

The role of HPV in cervical abnormalities, which range in severity from mild dysplasia to complete replacement of the cervical epithelium (CIS), has been studied extensively.

A positive correlation between grade of dysplasia (i.e., CIN I, II or III and CIS) and the presence of HPV has been established. Kurman et al., Am. J. Obstet. Gynecol., 159:293-296 (1988); Koutsky et al., Epidem. Rev., 10:122-163 (1988); deVilliers et al., The Lancet, 2:703-706 (1987). It has been determined that higher grade cytologic abnormalities are more often associated with HPV types 16, 18, 31 and 33 than with low-oncogenic HPV types. HPV types 6, 11, 16 and 18 are found in 80-90% of dysplasias. Koutsky et al., ibid.

However, HPV DNA also has been shown to be present in cervical samples evidencing no abnormality. For example, assays of cervical specimens for HPV DNA of types 6, 11, 16 and 18 by polymerase chain reaction (PCR) from 150 women with no cytologic abnormalities revealed that HPV DNA was present in 70-84% of the women. Young et al., British Med. J., 298(6665):11-14 (1989); Tidy et al., The Lancet, (Feb. 25, 1989) p. 434.

Relatively long-term epidemiologic studies which assess clinical progression from HPV-associated infections (koilocytosis and/or HPV DNA) to CIN I or greater suggest that 8-15% of HPV infections became more dysplastic with time. Syrjanen, K. et al. , Papillomaviruses: Cancer Cells, Vol. 5, Cold Spring Harbor, pp 281-288, (1987); de Brux et al., Bull Cancer (Paris), 70:410-422, (1983); Mitchell et al., The Lancet, 1:573-575, (1986); Syrjanen, K. et al., J. Cellular Biochem., Suppl., 13C, p.198 (1989). Conversely, this suggests that 85-92% of HPV infections do not progress with time. Progression was observed more frequently (about 35%) in individuals infected with HPV 16 than with types 6, 11, 18, 31, and 33 (7.5%-20%). It is unclear, at least, and unlikely, at best, that the presence of HPV DNA alone is a prognostic indicator of progression to serious dysplasias, at least in part because co-factors very likely play roles in disease progression.

Several lines of evidence suggest that expression of the E6 and/or E7 genes of HPV is necessary for oncogenesis. The expression of these genes causes transformation of rat or mouse epithelial or fibroblast cells (Crook et al., Proc. Nat'l. Acad. Sci., 85:8820-8824 (1988); Watanabe and Yoshiike, Int'l. J. Cancer, 41:896-900 (1988)), and, after insertion into a retroviral vector, produces tumors in nude mice. Yutsudo et al., Virol., 166:594-597 (1988). Phelps and co-workers showed that the E7 gene can cooperate with an activated ras oncogene to transform primary baby rat kidney cells and can transactivate a heterologous viral promoter. They also showed that portions of the predicted E7 protein are similar to several of the conserved domains of the adenovirus Ela protein, a transactivating protein which can also cooperate with an activated ras oncogene to transform rat epithelial cells. Furthermore, continued expression of the E7 gene seems to be required for maintenance of the transformed phenotype in cells which have been transformed by HPV 16 and EJ-ras Banks et al., J. Cellular Biochem., Suppl. 13C:202 (1988); Crook et al., EMBO Journal, 8(2):513-519 (1989). The E7 protein is localized in the cytoplasm and has been shown, by Western blot analysis, to be the most abundant HPV protein in cell lines containing HPV 16 DNA (CaSki and SiHa) or HPV 18 (HeLa, C4-1, and SW756). Seedorf et al., EMBO J., 6(1):139-144 (1987).

In all HPV-containing cervical carcinomas, and cell lines derived from them, the E6 and E7 genes are intact. Wilzynski et al., Virol., 166:624-627 (1988). Furthermore, transcripts of the E6 and/or E7 gene, but not necessarily other early genes, are consistently found in carcinomas and carcinoma cell lines. Baker et al., J. Virol., 6(4):962-971 (1987); Peter and Peter, Cancer Res., 48:324-328 (1988); Schneider-Gadicke and Schwarz, EMBO Journal, 5(9):2285-2292 (1986); Schwarz et al., In: Papillomaviruses and Human Disease, Springer-Verlag, pp. 443-466 (1987); Shirasawa et al., J. Gen. Virol., 68:583-591 (1987); Smotkin and Wettstein, Proc. Nat'l Acad. Sci., 83:4680-4684 (1986). The evidence suggests that expression of the E6 and/or E7 genes of HPV 16 and 18 are important for cellular transformation and may be important to the development of CIS.

Thus, although presently-available date suggest a strong association between HPV DNA in cervical samples and cervical abnormalities which range in severity from mild dysplasia to CIS, they also make it clear that many, and perhaps most, women in whom HPV DNA is present in cervical samples do not progress to dysplasia.

Patterns of transcription, including spliced transcripts, have been examined for several HPV types with high oncogenic potential. Examination of three cell lines containing HPV 18 has shown three patterns of transcription (ascertained by analysis of cDNA clones). Schwarz et al., Nature, 314:111-114 (1985). Pattern 1 contains full length transcripts of the E6 and E7 genes and the 5'-terminal 11 nucleotides (nts) of the E1 RNA (E1 starts at nt 914; splice donor site at nt 925) spliced to 3' cellular sequences. Patterns 2 and 3 contain shortened transcripts of E6 (intron of 182 nts missing between a splice donor site at nts 226-234 and the splice acceptor site at nts 401-412), designated E6*, which are joined to sequences of E7 and either downstream cellular sequences (Pattern 2) or to downstream HPV sequences. (Pattern 3). E6* splice sites also are present in DNA sequences of HPV 16, HPV 31, HPV 33, HPV 35, HPV 43, HPV 52 and HPV 56 but not in DNA of low-oncogenic HPV types (i.e., 6 and 11) Goldsborough et al., Virol., 171:306-311 (1989). Therefore, the E6* transcripts may be related to oncogenic potential of the HPV types. E6/E6* and E7 transcripts are found in HPV 16-containing cells, but they also contain spliced downstream E2-E4 exons, followed by cellular sequences. Two HPV 16 E6* transcripts have been identified in SiHa cells and two cancers. Smotkin et al, J. Virol. 63(3):1441-1447 (1989). Both of them use a common donor splice site at nts 224-232 and a splice acceptor site at either nts 399-417 (E6*I) or at nt 516-543 (E6*II). Because translation of E6*I terminates at a greater distance from the E7 translation start signal (AUG) than does translation termination of E6*II, the former transcript may allow more efficient translation of the E7 ORF.

Such transcription patterns described above form the basis for the design of probes end for their use in the method of detection and/or quantitation of specific transcripts of HPV in samples in the present invention. In addition, they provide the basis by which the present method is used for assessing risk of progression of cerival abnormalities. They provide the basis by which the present method is employed to detect and/or quantitate the above-described transcription patterns and/or other transcription patterns for assessing risk of progression of cervical abnormalities to more serious disease. As will be explained and illustrated, the present method is one in which RNA, DNA or the ratio of RNA to DNA of specific HPV types in cervical samples are detected and/or quantitated as a measure of neoplastic risk. Detection and/or quantitation of E6, E7 and spliced transripts of HPV types associated with oncogenes is or progression of cervical dysplasias as a means of determining neoplastic risk provides a novel tool which can be used not only in a diagnostic context (i.e., to assess presence or absence of risk of progression), but also in a therapeutic context (i.e., as a means by which treatment can be designed and/or monitored).

5

## Detection of Specific HPV Transcripts by the Present Method

The expression of the E6 and/or E7 genes, which can include downstream genes, and their expression relative to other genes is believed to be a prerequisite for HPV-related oncogenesis. In one embodiment of the present method, HPV transcription levels, particularly transcripts of at least a portion of the E6 and/or E7 genes of selected HPV types, are measured. That is, the amount of E6 and/or E7 mRNA present in a sample is detected and quantitated. Bicistronic and/or spliced transcripts of these genes also can be measured.

## The Method of the Present Invention

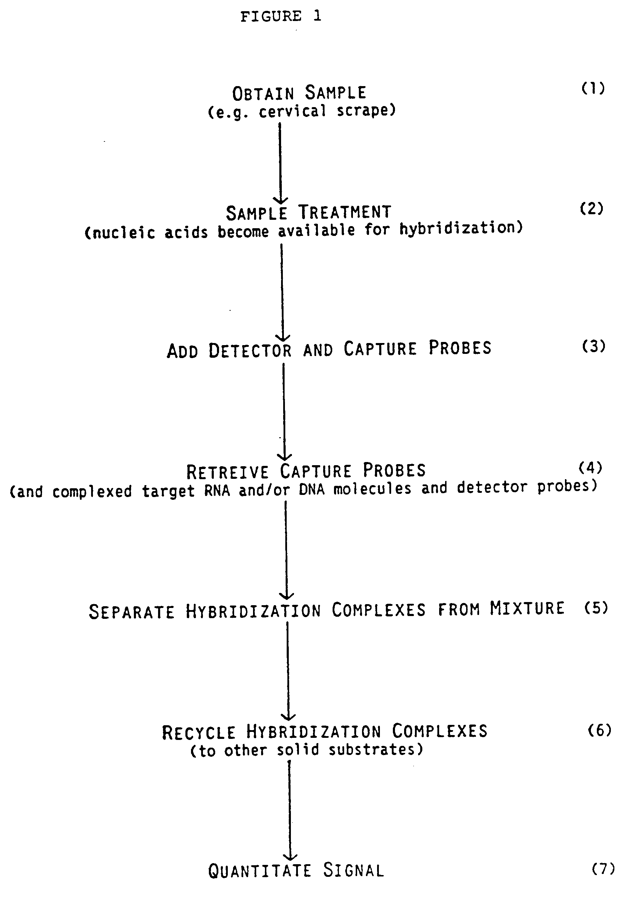

A method according to the invention is represented schematically in Figure 1 and is described below as it can be applied to any selected HPV type. It is further described, with reference to a specific HPV type (HPV 16). The sample to be assayed by the present method for E6/E7 and other spliced mRNA transcripts will generally be, for example, a cervical scrape, smear, mucus specimen, biopsy or tumor specimen. The sample does not have to be separated, filtered or precultured prior to being assayed by the present method. The sample can be mixed or diluted in a medium, if appropriate, and, if necessary, pretreated with an agent which disrupts cell and molecular structures within the cells. This disruption step frees the nucleic acids to be detected (the target nucleic acids). Cells can be disrupted, for example, using chaotropic agents which disrupt the molecular structure of a cell. That is, the agent denatures the secondary, tertiary and/or quaternary structures of biopolymers, including proteins, nucleic acids and polysacharides, which are generally found in biological specimens. Examples of chaotropic agents include chaotropic salts (e.g., guanidinium thiocyanate), hydrolytic enzymes (e.g., proteases, RNAases) and compounds that disrupt hydrophobic interactions (e.g., sodium dodecyl sulfate, phenols, dimethylformamide, dimethyl sulfoxide, tetramethyl urea or guanidinium hydrochloride). Physical or mechanical means of disrupting molecular structures, e.g., sonication, can also be used to release nucleic acids. If necessary, nucleic acids present in the cells and released from the cells are also treated to render them available for hybridization with complementary nucleic acid sequences (e.g., by heating to render double stranded sequences single stranded).

Two types of HPV type-specific probes are then contacted with the sample, either simultaneously or sequentially. One is the HPV type-specific tailed capture probes, which can be free or bound to a solid support. The capture probes are allowed to hybridize with the target transcripts in the sample mixture, forming a capture probe/target transcript complex. Either along with the capture probe or after the capture probe/ target transcript complex is formed, labeled detector probes are also contacted with the sample, which is maintained under appropriate conditions (e.g., time, temperature, pH, salt concentration, chaotrope concentration) for hybridization of complementary nucleotide sequences to occur and form a capture probe/target transcript/detector probe complex. The capture probe/target transcript/detector probe complex, which, for convenience, is subsequently referred to as the hybridization complex, is recovered from the sample mixture. This recovery is carried out by adding to the mixture containing hybridization complexes a solid substrate which is coated with a polynucleotide complementary to the tail of the capture probe. The tail of the capture probe present in the hybridization complex hybridizes with the complementary substrate, making it possible to separate the entire hybridization complex from the sample. In the case of pre-hybridized capture probes, the substrate containing the attached probe is removed from the sample mixture. Intact hybridization complexes may be released from the solid substrate by treatments which reversibly disrupt base pairings (the capture probe forms fewer base pairs with the solid substrate than do the probes with the target transcript) and complexes may be recaptured on to other solid substrates; the result of this cycling of hybridization complexes is reduction of noise which usually binds non-specifically to the solid substrate.

The quantity of detector probes and, thus, of the specific HPV transcript, present on the separated substrate can then be determined using known techniques, such is scintillation counting, densitometric scans of autoradiographs, fluorescence spectroscopy, and beta emission counters (e.g., Betagen). The method used will depend upon the detector probe label. The amount of signal is related to the amount of target nucleic acids captured, which in turn is proportional to the amount of transcript in the sample. The amount of transcript is related to the level of transcriptional activity of the selected gene (e.g., E6/E7 genes), which activity is indicative of the neoplastic risk. Thus, the method provides a sensitive assay for determining the risk of progression of HPV infection to serious CIN or CIS.

HPV-Specific Nucleic Acid Probes

Nucleic acid probes and probe sets which are specific for HPV genes associated with cervical cytologic abnormalities which often progress to more serious CIN or CIS, are used in the present method. Probes which are particularly useful in the present method are oligonucleotide probes having a nucleotide sequence which is complementary to at least a portion of the E6 and/or E7 ORF, or spliced portions thereof. A nucleotide sequence is complementary to a second or target nucleotide sequence (e.g., the E6 or E7 gene of HPV) if it hybridizes and remains hybridized to the second or target nucleotide sequence under the conditions used in the method.

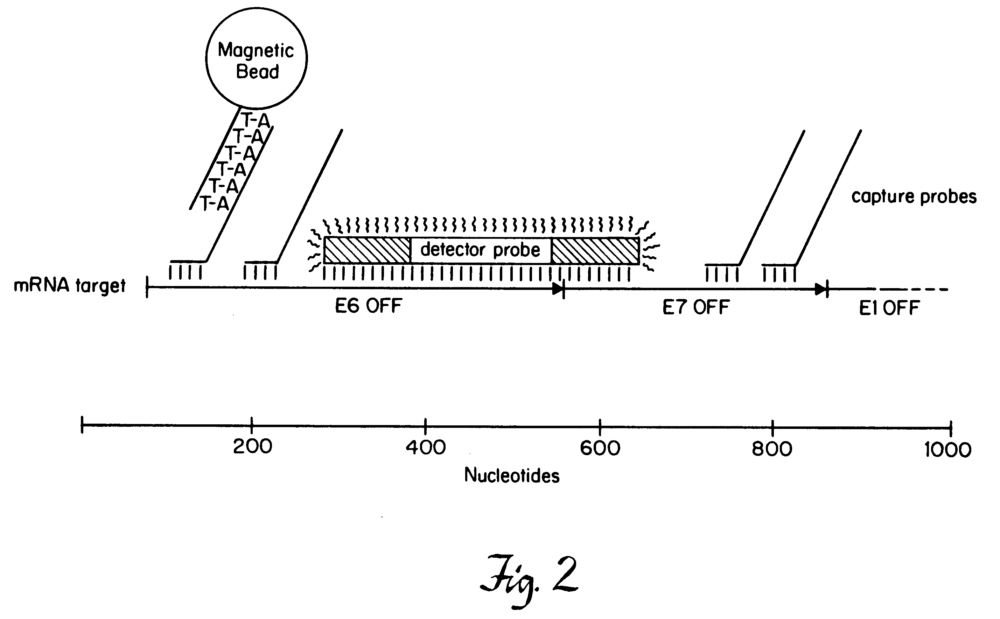

Two types of probes are used: tailed oligonucleotide "capture probes" and labelled "detector probes" (also referred to as labelled riboprobes). Tailed capture probes serve two purposes: they are complementary to/ hybridize to it least a portion of the ORF sequence of a gene of a selected HPV type or of the encoded transcript (mRNA) and they link the hybridization complex (capture probe/target nucleotide sequence/detector probe) to a solid support, thus making it possible to separate the hybridization complex from the remainder of the sample. A schematic representation of the complex of detector probe, target transcript, capture probes and solid support is shown in Figure 2. Labelled detector probes are generally single-stranded RNA or DNA probes which hybridize to portions of the bi- or polycistronic transcripts of genes of selected selected HPV types; specifically, they hybridize to portions of these genes which can be, but preferably are not also recognized by the capture probes. The selected HPV types are either high-oncogenic or low-oncogenic.

The capture probes are characterized by a polynucleotide tail which is generally formed from a nucleotide homopolymer, such as polydeoxyriboadenylate (poly(dA)), polydeoxyribocytidylate (poly(dC)), polydeoxyriboguanylate (poly (dC)), and polydeoxyribothymidylate (poly(dT)). The probe tail is complementary to a polynucleotide sequence which is affixed to a solid support, such as magnetic beads, polysyrene beads and polystyrene dipsticks, allowing the probe to be captured and separated from the test sample.

Probes particularly useful in the present invention as capture probes have the following characteristics:

1. They are synthesized easily by in vitro transcription (for detector probe) or by chemical means (for oligonucleotide capture probes). Detector probes should be easily labeled, and to high specific activities, during in vitro transcription or chemical synthesis or by post-synthetic modification with either isotopic or non-isotopic markers which can be easily quantitated.

2. They are HPV type-specific. Although HPV 6 sometimes has been associated with carcinomas, it and HPV 11 most often are associated with low risk for progression to high grade dysplasia. Because E6 and E7 genes of HPV 6 and HPV 11 show high sequence conservation, probes to these regions may cross-hybridize; this result would not detract from the efficacy of the assay. Probes which are particularly useful in the present assays are those specific for HPV 16, 18, 31, 33 and 35.

3. They are of sufficient length and nucleotide sequence that they do not dissociate from specific target sequences on viral transcripts in the assay format, and hybridize specifically and quantitatively to transcripts of HPV.

4. They hybridize to selected HPV DNA sequences or transcripts of selected types of HPV. Target transcripts contain at least a portion of the ORF to allow for stable hybridization of the detector probes and at least one capture probe. Probes are designed in such a manner that efficiency of hybridization of probes to target transcripts is not diminished by excision of introns from transcripts (e.g., formation of E6*I and E6*II).

5. The integrity of probes used is maintained in the conditions of the assay.

Oligonucleotide probes which are particularly useful as capture probes in the present invention are poly-(dA)-tailed oligonucleotide probes, such as those prepared according to the method described by M.L. Collins In European Patent Application 0265244, filed October 21, 1987. Capture probes which are prehybridized to a solid substrate also can be used. Prehybridised probes are described in detail by by M.L. Collins and D.V. Morrissey, in published PCT application PCT/US 90/01205, filed March 6, 1990.

The second type of probe used in the present method is a riboprobe or detector probe whose sequence is specific for an HPV transcript or selected HPV DNA, and which generally have a detectable label, such as a radionuclide, or fluorescent indicator. Detector probes are used to quantitate the DNA or mRNA. Detector probes can be prepared by art recognized techniques. Detector probes are generally single stranded RNA probes which hybridize to bi- or polycistronic transcripts of a selected HPV gene, such as the E6 or E7 gene. The detector probes are preferably labeled with a radionuclide, such as $^{32}$P or $^{125}$I, but can be labeled by any means which does not interfere with the ability of the detector probes to hybridize to complementary sequences and which can be detected using available techniques.

7

The present assay system and method of using it can be incorporated into a kit for clinical use. Such a kit includes a sample processing solution containing a chelating agent, such as EDTA, and an agent for disrupting molecular structures (e.g., membranes) of cells (e.g., a chaotropic agent); the tailed capture probes and labelled detector probes; at least one buffer; an agent for inhibiting RNAase enzymes (to prevent degradation of the target transcripts); a solid support (e.g., magnetic heads or polystyrene substrate) coated with a polynucleotide which is complementary to the capture probe tail; reference or standard nucleic acids to be run simultaneously with the sample as a control; and a wash buffer containing a detergent and an agent (e.g., a chaotropic agent) for disrupting molecular structures of cells. The kit can optionally contain additional wash buffers, a means for detecting the labelled detector probe, one or more elution buffers, amplification or cloning reagents and/or additional positive control samples or negative control sample. Amplification of the target sequences, if desired, can be accomplished, for example, by the technique described by Mullis in U.S. Patent 4,683,202. Amplification of the detector probe, after it has been cycled with the hybridization complexes, and/or cloning of the target sequences, can be accomplished, for example, by the method described by Maniatis et al. in Molecular Cloning: A Laboratory Manual, Cold Spring Harbor Laboratory, Cold Spring Harbor, NY (1982).

The invention will now be further illustrated by the following Exemplification.

EXEMPLIFICATION

Design, Preparation, and Physical Description of Probes

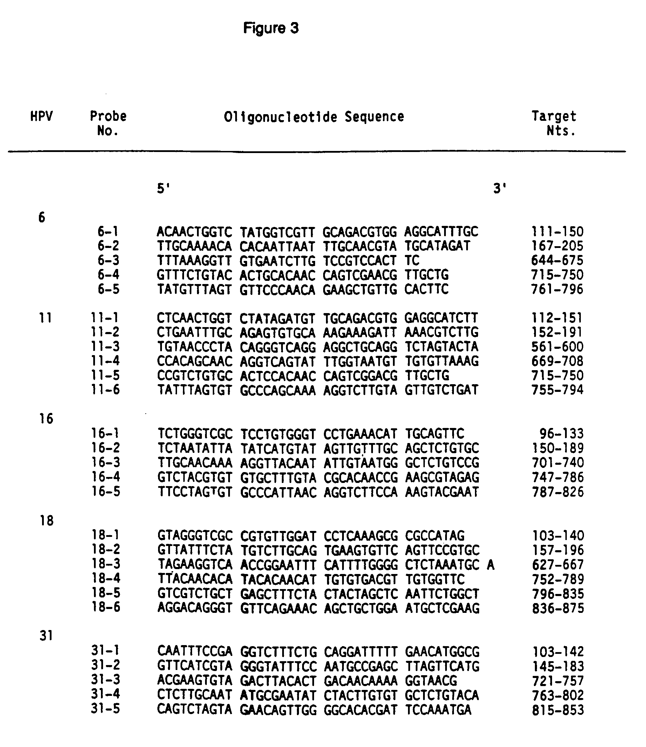

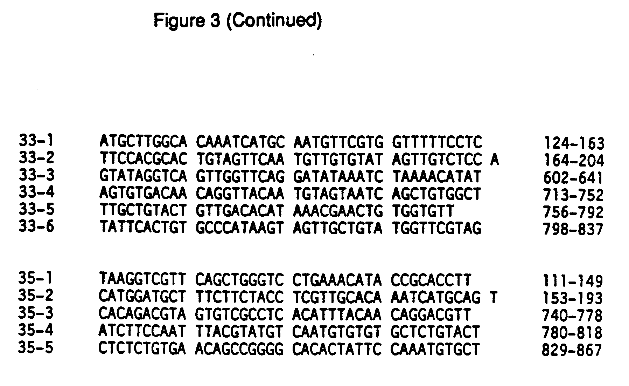

HPV type-specific oligonucleotide capture probes, about 4O nucleotides long are shown in Figure 3. The Wordsearch/Segments Programs of the University of Wisconsin Suite was used to identify regions of HPV genomic, sense strands (i.e., RNA sense) which might hybridize to these 33 oligonucleotide probes, and results of these analyses are shown in the Table.

Table.  Predicted Number of Base Pairs
Between Oligonucleotide Probes and
Discreet Regions of HPV Genomes

| HPV Type | Probe No. | Probe Length | ORF | Nts Recognized By Probe | 6 | 11 | 16 | 18 | 31 | 33 | 35 |
|---|---|---|---|---|---|---|---|---|---|---|---|
| **6** | | | | | | | | | | | |
| | 6-1 | 40 | E6 | 111-150 | 40 | 34 | <20 | NF | 24/4 | <20 | NF |
| | 6-2 | 39 | E6 | 167-205 | 39 | 30 | 23/8 | NF | 25/1 | 22/6 | NF |
| | 6-3 | 32 | E7 | 644-675 | 32 | 25 | 23/6 | <20 | <20 | NF | NF |
| | 6-4 | 36 | E7 | 715-750 | 36 | 30 | 21/4 | 22/1 | <20 | NF | NF |
| | 6-5 | 36 | E7 | 761-796 | 36 | 21/10 | NF | NF | 22 | NF | NF |
| **11** | | | | | | | | | | | |
| | 11-1 | 40 | E6 | 112-151 | 34 | 40 | <20 | <20 | <20 | NF | NF |
| | 11-2 | 40 | E6 | 152-191 | 33 | 40 | NF | NF | 22/1 | 22/8 | NF |
| | 11-3 | 40 | E7 | 561-600 | 36 | 40 | <20 | <20 | 24/2 | 20/3 | NF |
| | 11-4 | 40 | E7 | 669-708 | 37 | 40 | NF | 23/4 | <20 | <20 | NF |
| | 11-5 | 36 | E7 | 715-750 | 30 | 36 | <20 | 23/8 | <20 | NF | NF |
| | 11-6 | 40 | E7 | 755-794 | 31 | 40 | 20 | 25/2 | <20 | NF | NF |
| **16** | | | | | | | | | | | |
| | 16-1 | 38 | E6 | 96-133 | <20 | 23 | 38 | NF | <20 | NF | 22 |
| | 16-2 | 40 | E6 | 150-189 | <20 | NF | 40 | <20 | 22/3 | 20/1 | NF |
| | 16-3 | 40 | E7 | 701-740 | <20 | <20 | 40 | 21/4 | 32 | 29 | 30 |
| | 16-4 | 40 | E7 | 747-786 | <20 | <20 | 40 | 21/4 | <20 | <20 | NF |
| | 16-5 | 40 | E7 | 787-826 | 26/1 | 20 | 40 | <20 | 32 | 22/2 | NF |
| **18** | | | | | | | | | | | |
| | 18-1 | 38 | E6 | 103-140 | NF | NF | <20 | 38 | <20 | NF | NF |
| | 18-2 | 40 | E6 | 157-196 | 21/3 | <20 | NF | 40 | 23/3 | <20 | NF |
| | 18-3 | 41 | E7 | 627-667 | NF | NF | <20 | 41 | 23/2 | 20/4 | NF |
| | 18-4 | 38 | E7 | 752-789 | <20 | <20 | <20 | 38 | <20 | <20 | NF |
| | 18-5 | 40 | E7 | 796-835 | 25 | <20 | 27/3 | 40 | <20 | NF | NF |
| | 18-6 | 40 | E7 | 836-875 | <20 | <20 | NF | 40 | 20 | NF | NF |
| **31** | | | | | | | | | | | |
| | 31-1 | 40 | E6 | 103-142 | 23 | 24/4 | NF | 21/3 | 40 | NF | NF |
| | 31-2 | 40 | E6 | 145-183 | <20 | <20 | <20 | <20 | 40 | <20 | NF |
| | 31-3 | 37 | E7 | 721-757 | NF | <20 | 21/2 | NF | 37 | <20 | NF |
| | 31-4 | 40 | E7 | 763-802 | <20 | NF | <20 | NF | 40 | NF | 31 |
| | 31-5 | 39 | E7 | 815-853 | NF | NF | 28 | <20 | 39 | 20 | 28 |
| **33** | | | | | | | | | | | |
| | 33-1 | 40 | E6 | 124-163 | NF | <20 | 22 | 21/1 | 26 | 40 | 24 |
| | 33-2 | 41 | E6 | 164-204 | NF | 20/2 | <20 | 20/2 | <20 | 41 | 22/2 |
| | 33-3 | 40 | E7 | 602-641 | <20 | 30/3 | 30 | <20 | 31 | 40 | 33 |
| | 33-4 | 40 | E7 | 713-752 | <20 | 24/8 | NF | NF | 26/3 | 40 | NF |
| | 33-5 | 37 | E7 | 756-792 | 20/4 | <20 | <20 | NF | 23/3 | 37 | NF |
| | 33-6 | 40 | E7 | 798-837 | 27/6 | 24/2 | 22/6 | <20 | 21/2 | 40 | 25/2 |
| **35** | | | | | | | | | | | |
| | 35-1 | 39 | E6 | 111-149 | NF | NF | 22 | NF | NF | 20 | 39 |
| | 35-2 | 41 | E6 | 153-193 | NF | NF | NF | NF | NF | 26 | 41 |
| | 35-3 | 39 | E7 | 740-778 | NF | NF | NF | NF | NF | NF | 39 |
| | 35-4 | 39 | E7 | 780-818 | NF | NF | 32 | NF | 30 | NF | 39 |
| | 35-5 | 39 | E7 | 829-867 | NF | NF | 31 | NF | 30 | NF | 39 |

Numbers in the Table refer to the base pairs in a discreet region of the viral genome which are predicted, from computer analyses (algorithm of Wilbur and Lipman; P.N.A.S. 80:726-739, 1983)), to pair with portions of the oligonucleotides. Numbers after slash marks (/) indicate the gaps that would be needed in the oligonucleotide or target sequence to allow for the number of base pairs. For each HPV type, the nucleotide sequences (GenBank designations) that are covered by each type-specific oligonucleotide are shown. In some cases, the computer program did not find any likely regions of significant base pairing between the oligonucleotide sequences and genomic sequences; these cases are noted "not found" (NF). The oligonucleotides in Table 1 are unlikely to form stable hybrids with other types of HPV, therefore, they are considered to be type-specific.

## Design of Exon-Specific Capture Probes for HPV 16, HPV 18, HPV 31, HPV 33 and HPV 35

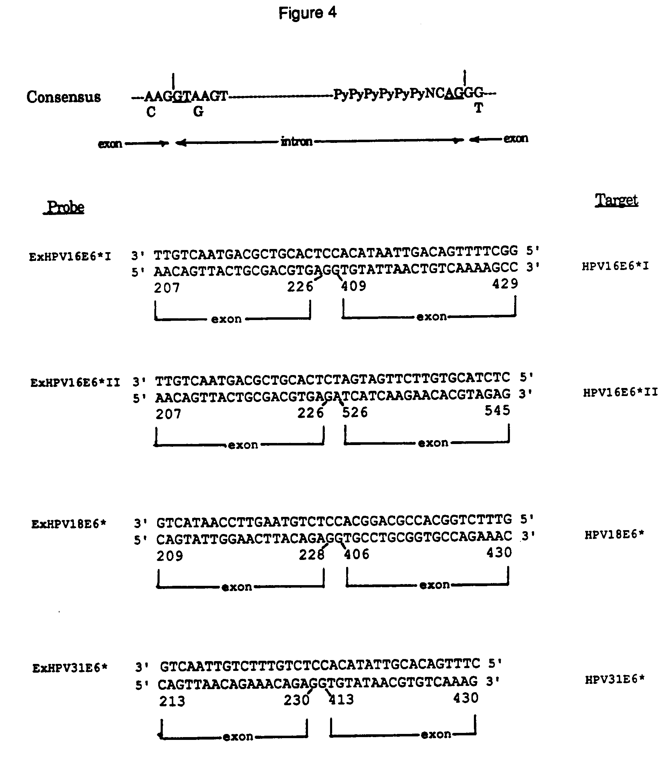

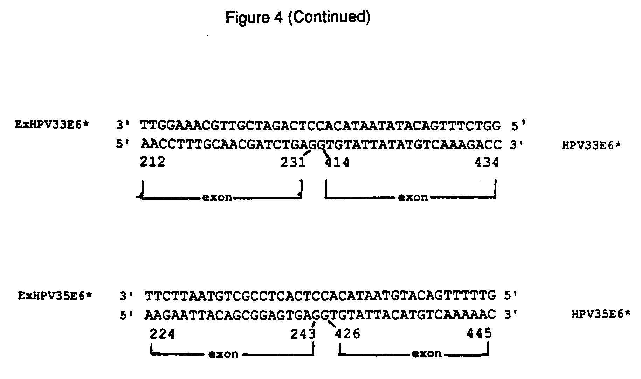

Splice junction sequences in the E6 ORF have been identified in several oncogenic types of HPV, including HPV 16, HPV 18, HPV 31, HPV 33, and HPV 35 but they are not present in the E6 ORF's of HPV 6 and HPV 11. These splice donor and acceptor sites are utilized in HPV 16 and HPV 18, as is demonstrated by the presence of spliced mRNA's (determined by sequencing cDNA's) in carcinoma cell lines. The presence of spliced transcripts can be used as predictors of progression to more serious disease. Thus, splice-junction capture probes which are predicted to form stable hybrids only with spliced transcripts were designed. Approximately 20 nucleotides of each capture probe hybridize to the 3' end of the upstream exon and the remaining 20 nucleotides hybridize to the 5' end of the downstream exon. Sequences of these probes and their target sites on transcripts are shown in Figure 4. Analyses of the alignment (algorithm of Wilbur and Lipman) of splice-junction probes and the genomes of HPV 6, HPV 11, HPV 16, HPV 18, HPV 31, and HPV 33 revealed that each probe is predicted to hybridize only to the transcripts of the HPV type for which the probe was designed. Different probes were designed for detection of each of the two E6 spliced mRNA's (E6*I and E6*II) of HPV 16.

## Design of HPV 16-Specific Capture Probes and Arrangement of Probes in the HPV 16 Assay

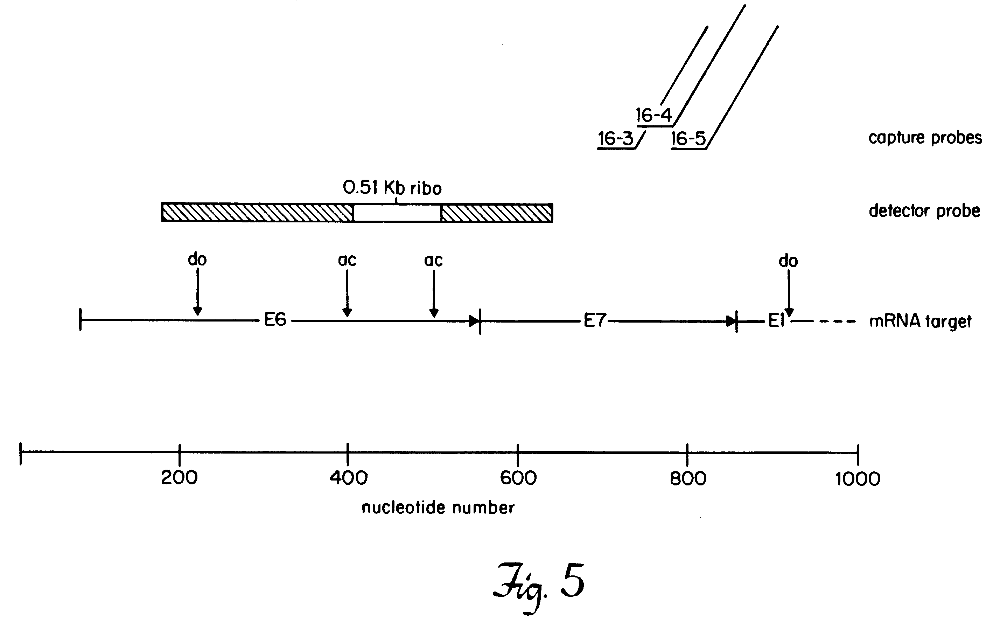

Ten HPV 16-specific oligonucleotide probes (about 40 nucleotides long) were designed and tested in the assay, described below. Three of these probes, designated 16-3, 16-4, and 16-5, were adapted for use in the assay because they afforded high sensitivity and specificity. The placement of these three probes with respect to the RNA target and the detector probes is shown in Figure 5. Capture probe 16-3, the probe closest to the detector probe, hybridizes to target sequences 46 nucleotides beyond the 3' end of the hybridization site covered by the detector riboprobe. Therefore, after hybridization of the detector probe to mRNA, only a short portion remains single stranded (i.e., not covered by capture or detector probes), thus minimizing the chances for degradation by RNases.

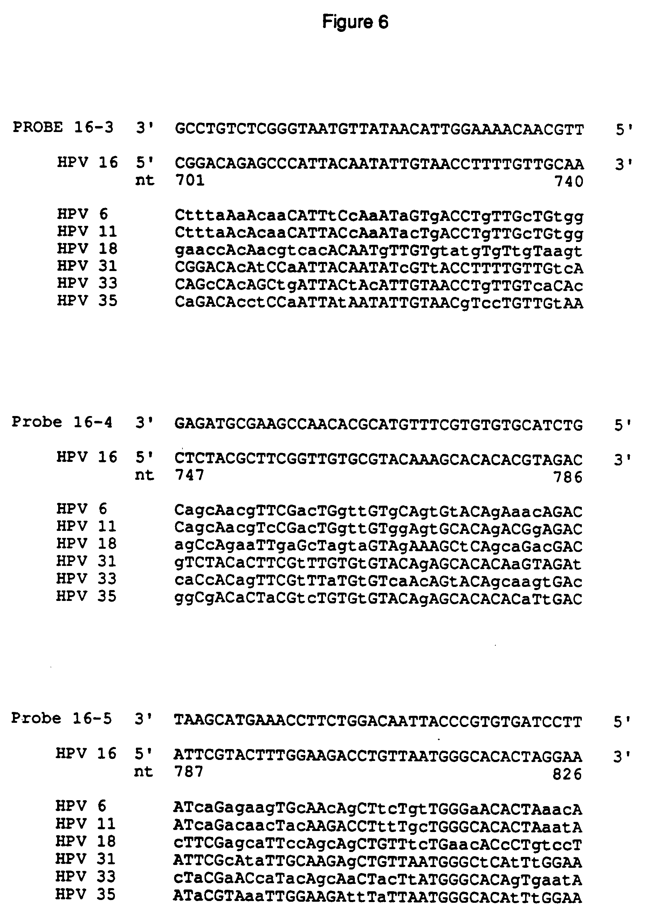

The alignment of these capture probes with sequences in HPV 6, HPV 11, HPV 16, HPV 18, HPV 31, HPV 33 and HPV 35 is shown in Figure 6. Inspection of potential base pairs between these probes and the other genital HPV types, an alignment that was made by visual and computer analyses, reveals that base mismatches (not underlined) are numerous and approximately evenly distributed along the capture probes. As a result, these hybrids would be very unstable.

The 3' ends of capture probes are extended by sequential addition of d(A) residues with terminal transferase such that the final poly-d(A) "tail" is 150-200 nucleotide long, the minimum length that will allow unencumbered capture of the hybridization complex to d(T)-modified magnetic beads.

## Design and Preparation of HPV 16-Specific Detector Riboprobe and Target RNA

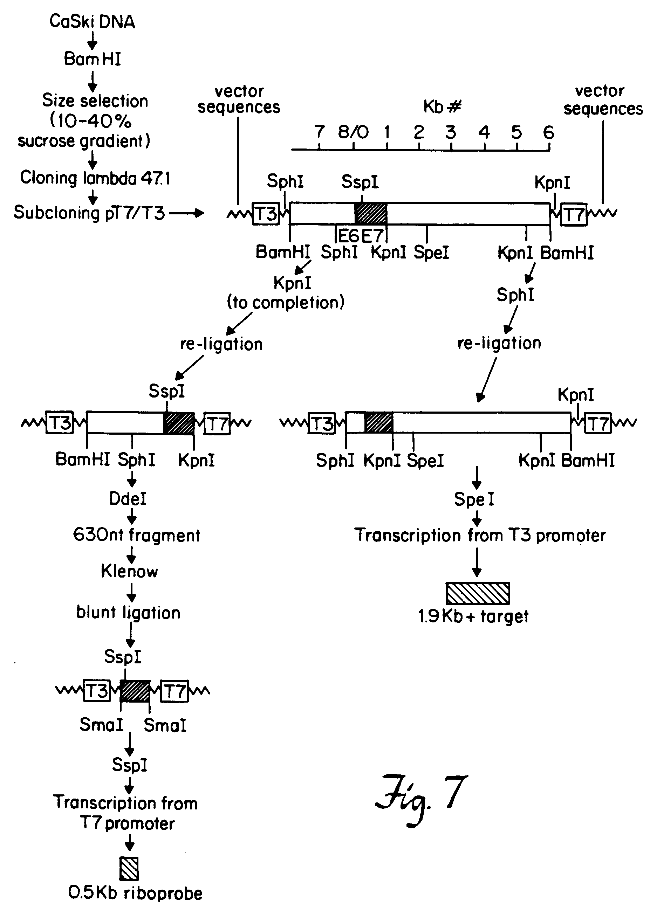

HPV 16 DNA was cloned from CaSki cells, known to contain HPV 16 genomes (Baker et al., J. Virol., 61(4): 962-971, 1987), into lambda 47.1 from which it was subcloned into the pT7/T3α-18 plasmid (Bethesda Research Laboratory). A summary of the cloning/subcloning scheme is shown in Figure 7. A 630 nucleotide Dde I fragment (nucleotides 25-655; Seedorf et al., Virol., 145:181-185, 1985) was excised, end-filled, and subcloned into Sma I-restricted pTf/T3α-18 plasmid. Sequence analysis, obtained by double or triple reads in most cases, revealed that the insert contained only nts 25-655 of the HPV 16 genome. In vitro transcription with T7 polymerase on this construct, linearized with Ssp I restriction endonuclease, results in a single product with a homogeneous electrophoretic mobility and an apparent size of 0.5 Kb, that should hybridize to the 3' three-fourths of the E6 ORF and the 5' third of the E7 ORF. The detector probe contains, in addition to 471 nucleotides of the HPV 16 sequence, 22 additional nucleotides of the plasmid sequence on its 5' end, a result of transcription initiation on the T7 promoter and procession through a portion of the multiple cloning site in the vector. These additional 5' nucleotides will not interfere with hybridization of the capture probes, the most proximal being 46 nucleotides away. The detector probe, after hybridization to target mRNA, should extend about 4O nucleotides 5' to the splice donor site at nucleotides 224-232 (Figure 5); this hybrid is predicted to be very stable. Excision of either of the E6 introns would result in mRNA molecules that base pair with either 162 or 288 nucleotides of the detector probes. These hybrids should be stable in the assay. Following the manufacturers' instructions for synthesis of transcripts with T7 polymerase, $^{32}$P-labeled riboprobes with specific activities of 5 x 10$^8$ cpm/μg are produced. Placement of the detector probe on E6/E7 transcripts was designed such that E6/E7 target sequences flanking the hybridization site of the detector riboprobe would be available for hybridization to capture probes.

The sensitivity of the HPV 16 assay was also determined. For this purpose it was important to produce, by in vitro transcription, well characterized positive targets (i.e., mRNA-like molecules which contain the ORF's of the E6 and E7 genes). Transcription from the T3 promoter in the construct which contained full length genomic HPV 16, revealed a putative transcription terminator just upstream from the E6 and E7 genes (nucleotides 7400-7600 in the non-coding region). Visual inspection of the sequences in this region, which lies just downstream from a polyadenylation signal, suggests that transcription of plus-sense RNA from this portion of the genome would produce RNA's which have numerous stretches of U residues, characteristics of transcription terminator; therefore, a 1.3 Kb Sph I fragment which includes about half of the region rich in termination sequences and a few sequences in the vector was deleted. Transcription from the resulting construct (linearized with Spe I) with T3 polymerase yields one expected major transcript of 1.9 Kb as well as a shorter minor RNA species (about 600 nucleotides shorter than the major one). The 1.9 Kb transcript was used as a positive target in the assay.

Design and Performance of the HPV 16 Assay

Preparation of samples and procedures for performance of the assay are the same as those described by M. Collins In European Patent Application, Number 0265244, the teachings of which are incorporated here in by reference. In brief, cells from cervical specimens (e.g., cervical lavage, scrapes, bioposies, and tumors) are mixed with sample processing buffer (SPB), containing 5M guanidinium isothiocynate (GuSCN) and 0.1M ethylenediaminetetraacetic acid (EDTA) in order to solubilize the cells and sample matrix and to inhibit RNAses. Samples are diluted such that the final concentration of GuSCN is 2.5M and incubated with 67 ng/ml of each capture probe and $1.4 \times 10^7$ cpm/ml of detector probe at 37° for 2-18 hours. Then, d(T)-coated magnetic beads are added (final concentration of 0.125% solids) and the resulting mixture is incubated at 37° for 15 minutes; d(A)-tailed capture probes hybridize to the d(T)-coated magnetic beads during this period. Wash buffer (0.5 M GuSCN, 40 mMTris-HCl, pH 7.8, 10 m MEDTA, 0.5% sarkosyl, 0.2% bovine serum albumin, and 0.1% antifoam) is added to the mixture and the magnetic beads then are concentrated on the walls of the tubes by a magnetic field. The supernatant fluid is aspirated. Beads are washed two additional times with wash buffer, following the procedure just described, and then re-suspended in a buffer containing 3.25M GuSCN, 100mM Tris-HCl, pH 7.8, 65mM EDTA, 0.5% BSA, and 0.5% sarkosyl. Following incubation of the suspension at 37° for 10 minutes, nucleic acid complexes should be released from the beads due to disruption of the A-T bonds between the poly-d(A) tails of the capture probes and the oligo-d(T) chains of the magnetic beads. After concentration of the magnetic beads on the tube walls by a magnetic field, the release buffer and nucleic acid complexes are transferred to another tube and hybridization complexes are captured on a second set of magnetic beads, following the procedure just described. This capture, release, and re-capture of the nucleic acid complexes on magnetic beads is called reversible target capture.

A second round of reversible target capture is performed and nucleic acid complexes in the release buffer are captured on a third set of beads. After the beads are washed once, they are resuspended in wash buffer and an aliquot of the suspension is applied to a nitrocellulose membrane. Radioactivity can be detected by exposure of the membrane to film or by quantitation of counts on a beta emission blot analyzer (e.g., Betagen). Exposure of the film may be quantitated on a densitometer. Alternatively, fluorescein-labeled detector oligonucleotides or riboprobes may be used in the assay.

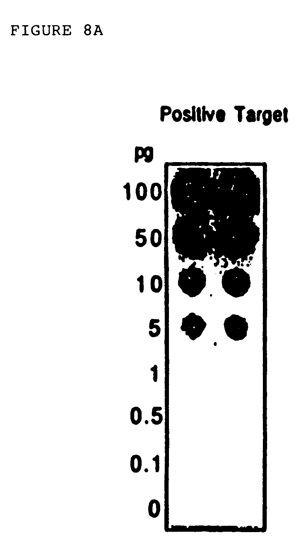

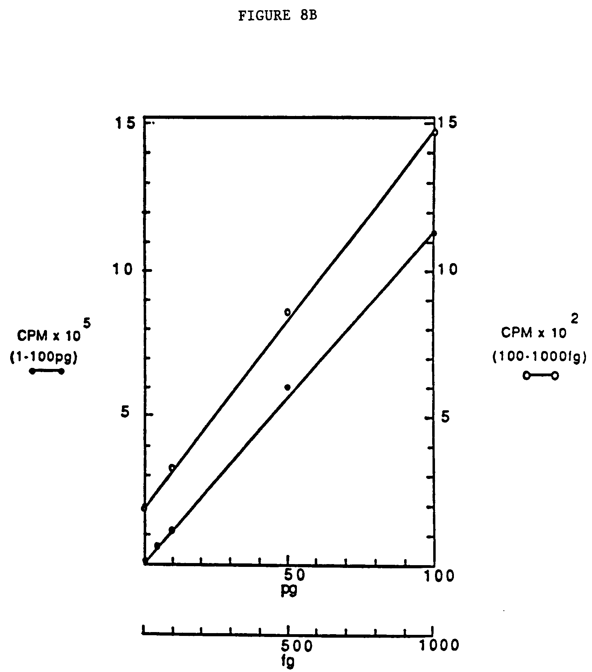

Efforts were made to quantitate the sensitivity of the HPV 16 assay using in vitro-generated positive target transcripts. Examination of the X- ray film revealed (Figure 8A) that 500 fg of target (500,000 molecules) could be visualized and that a signal was not detected if target RNA was omitted; no signal was detected if lysates from 200,000 HeLa cells (which contain HPV 18 sequences) are used in the assay. A standard curve, produced by plotting radioactivity on beads vs. amount of positive target RNA, is a straight line for RNA target values of 100 fg to 100 pg (Figure 8B).

## Claims

1. A method of determining the prognosis of individuals infected with HPV comprising the steps of; (1) measuring the level of HPV activity by detecting transcripts of all or a portion of the E6 and/or E7 HPV genes (and/or spliced transcripts thereof) in a sample e.g. a cervical sample, and (2) comparing the measurements of HPV activity with a previously established relationship between activity and risk of progression to serious cervical dysplasia or carcinoma.

2. A method according to claim 1 wherein step (1) further comprises the step of determining the RNA/DNA ratios of the E6 and/or E7 HPV genes.

3. A method according to claim 1 or claim 2 wherein the HPV activity measured is that of at least one high-oncogenic type HPV, e.g. high oncogenic types HPV16, HPV18, HPV31, HPV33 and HPV35.

4. A method according to any one of claims 1-3 wherein the E6 and/or E7 transcript further comprises nucleotide sequences downstream of the E7 gene.

5. A method according to any one of the preceding claims wherein the transcripts are detected by use of a probe or probes, for example at least one tailed capture probe which is complementary to a transcript of at least a portion of the E6 or E7 gene of the HPV and which has a polynucleotide (e.g. poly d(A) poly d(T)) tail and a labelled (for example radionuclide e.g. 32P or 125I-labelled) detector probe which is complementary to a different portion of the transcript of the E6 or E7 gene of the HPV, further comprising the steps of; (a) mixing the capture and detector probes with a sample (e.g. a cervical sample) containing target HPV transcripts to form a capture probe/target transcript/detector probe hybridization complex, (b) contacting the complex with a solid support (for example paramagnetic beads, polystyrene beads and polystyrene dipsticks having affixed thereto a polynucleotide e.g. poly d-(T) and poly d(A), which polynucleotide is complementary to the polynucleotide tail of the capture probe present in the hydridization complex), under conditions appropriate for hybridization of complementary nucleotide sequences to occur, thereby forming a hybridization complex /solid support complex, (c) separating the hybridization complex/solid support complex from the product of step (c) by removing the solid support; and (d) detecting the hybridization complex/solid support complex.

6. A method according to claim 5 wherein the capture probe is prehybridized with the polynucleotide affixed to the solid support.

7. A method according to claim 5 or claim 6 wherein the HPV is selected from high oncogenic types HPV16, HPV18, HPV31, HPV33 and HPV35 and the transcripts are detected by use of a probe or probes consisting essentially of a nucleotide sequence selected from the sequences of probes 16-1, 16-2, 16-3, 16-4, 16-5, 18-1, 18-2, 18-3, 18-4, 18-5, 18-6, 31-1, 31-2, 31-3, 31-4, 31-5, 33-1, 33-2, 33-3, 33-4, 33-5, 33-6, 35-1, 35-2, 35-3, 35-4, 35-5, ExHPV16E6*I, ExHPV16E6*II, ExHPV18E6*, ExHPV31E6*, ExHPV33E6* and ExHPV35E6*, as described in Figures 3 and 4.

8. A method according to any one of the preceding claims further comprising the step of determining the pattern of transcription of the E6 and/or E7 genes (and/or spliced transcripts thereof) by using a set of probes comprising at least two probes each being complementary to a different target HPV transcript and wherein the extent of hybridization of each of the probes to each of the transcripts is quantitated to determine the pattern of transcription.

9. A method according to any one of the preceding claims for determining the risk of progression to serious dysplasia of the cervix or to cervical carcinoma in a female in whom HPV is present in the cervix, further comprising the step of comparing the measured HPV activity with that associated with; (i) high grade dysplasia or carcinoma in situ, or (ii) the risk of progression to serious dysplasia of the cervix or to cervical carcinoma, wherein similarity as determined from the comparison is indicative of the risk of progression to serious dysplasia of the cervix or to cervical carcinoma.

**Patentansprüche**

1. Ein Verfahren zum Bestimmen der Prognose HPV infizierter Personen, das die Schritte umfaßt: (1) Messen des HPV-Aktivitätsspiegels mittels Detektion von Transkripten der gesamten oder eines Teils der E6 und/oder E7 HPV-Gene (und/oder deren gespleißten Transkripte) in einer Probe, z. B. einem Gebärmutterhals-Abstrich, und (2) Vergleich der HPV-Aktivitätsmessungen mit einer zuvor festgelegten Relation zwischen Aktivität und Progressionsrisiko einer schweren Gebärmutterhalsdysplasie oder eines -karzinoms.

2. Ein Verfahren nach Anspruch 1, worin Schritt (1) des weiteren den Schritt der Bestimmung des RNA/DNA Verhältnisses der E6 und/oder E7 HPV-Gene umfaßt.

3. Ein Verfahren nach Anspruch 1 oder Anspruch 2, worin die HPV-Aktivität von mindestens einem hoch onkogenen HPV-Typ, z. B. den hoch onkogenen Typen HPV16, HPV18, HPV31, HPV33 und HPV35, gemessen wird.

4. Ein Verfahren nach einem der Ansprüche 1 bis 3, worin das E6 und/oder E7 Transkript des weiteren Nucleotid-Sequenzen downstream des E7 Gens umfaßt.

5. Ein Verfahren nach einem der vorausgehenden Ansprüche, worin die Transkripte mittels Verwendung einer Sonde oder Sonden detektiert werden, beispielsweise mindestens einer mit Schwanz versehenen Einfang-Sonde, die komplementär zu einem Transkript von wenigstens einem Teil des E6 oder E7 Gens des HPV ist und einen Polynucleotid-(z. B. Poly d(A) Poly d(T))-Schwanz besitzt, und einer markierten (beispielsweise Radionuklid, z. B. 32P oder 125I, markierten) Detektor-Sonde, die komplementär zu einem anderen Teil des Transkripts des E6 oder E7 Gens des HPV ist, wobei das Verfahren des weiteren die Schritte umfaßt: (a) Mischen der Einfang- und Detektor-Sonden mit einer die Ziel-HPV-Transkripte enthaltenden Probe (z. B. Gebärmutterhals-Abstrich), um einen Einfang-Sonde/Zieltranskript/Detektor-Sonde Hybridisierungskomplex zu bilden, (b) In-Kontakt-Bringen des Komplexes mit einem festen Träger (zum Beispiel paramagnetischen Kügelchen, Polystyrol-Kügelchen und Polystyrol-Tauchstäbe, die außerdem ein haftendes Polynucleotid besitzen, z. B. Poly d(T) und Poly d-(A), wobei dieses Polynucleotid komplementär zum Polynucleotidschwanz der im Hybridisierungskomplex vorhandenen Einfang-Sonde ist) unter Bedingungen, die für eine sich zu ereignende Hybridisierung der komplementären Nucleotidsequenzen geeignet sind, und auf diese Weise einen Hybridisierungskomplex/fester Träger Komplex bilden, (c) Trennen des Hybridisierungskomplex/fester Träger Komplexes vom Produkt des Schrittes (c) durch Entfernen des festen Trägers; und (d) Detektion des Hybridisierungskomplex/fester Träger Komplexes.

6. Ein Verfahren nach Anspruch 5, worin die Einfang-Sonde mit dem am festen Träger haftenden Polynucleotid vorhybridisiert ist.

7. Ein Verfahren nach Anspruch 5 oder Anspruch 6, worin HPV aus den hoch onkogenen Typen HPV16, HPV18, HPV31, HPV33 und HPV35 gewählt ist und die Transkripte mittels Verwendung einer Sonde oder Sonden detektiert werden, die im wesentlichen aus einer Nucleotid-Sequenz bestehen, die aus den Sequenzen der Sonden 16-1, 16-2, 16-3, 16-4, 16-5, 18-1, 18-2, 18-3, 18-4, 18-5, 18-6, 31-1, 31-2, 31-3, 31-4, 31-5, 33-1, 33-2, 33-3, 33-4, 33-5, 33-6, 35-1, 35-2, 35-3, 35-4, 35-5, ExHPV16E6*I, ExHPV16E6*II, ExHPV18E6*, ExHPV31E6*, ExHPV33E6* und ExHPV35E6*, wie in den Abbildungen 3 und 4 beschrieben, gewählt ist.

8. Ein Verfahren nach einem der vorausgehenden Ansprüche, das des weiteren den Schritt des Bestimmens des Transkriptionsmusters der E6 und/oder E7-Gene (und/oder deren gespleißte Transkripte) mittels Verwendung einer Reihe von Sonden umfaßt, die mindestens zwei Sonden umfaßt, wobei jede Sonde zu einem anderen Ziel-HPV-Transkript komplementär ist, und worin das Ausmaß der Hybridisierung einer jeden Sonde mit jedem der Transkripte quantifiziert wird, um das Transkriptionsmuster zu bestimmen.

9. Ein Verfahren nach einem der vorausgehenden Ansprüche zum Bestimmen des Progressionsrisikos einer schweren Gebärmutterhalsdysplasie oder eines Gebärmutterhalskarzinoms einer Frau, deren Gebärmutterhals HPV infiziert ist, das des weiteren den Schritt des Vergleichs der gemessenen HPV-Aktivität mit jener Aktivität umfaßt, die verbunden ist mit: (i) hochgradiger Dysplasie oder Karzinom in situ, oder (ii) dem Progressionsrisiko einer schweren Gebärmutterhalsdysplasie oder eines Gebärmutterhalskarzinoms, worin die aus dem Vergleich bestimmte Ähnlichkeit das Progressionsrisiko einer schweren Gebärmutterhalsdysplasie oder eines Gebärmutterhalskarzinoms aufzeigt.

**Revendications**

1. Procédé de détermination de pronostic pour des individus infectés par le VPH comprenant les étapes de : (1) mesure du niveau d'activité du VPH par détection des transcripts de la totalité ou d'une portion des gènes E6 et/ou E7 (et/ou des transcripts épissés de ceux-ci) dans un échantillon, par exemple un échantillon cervical, et (2) comparaison des mesures d'activité de VPH à l'aide d'une relation préalablement établie entre l'activité et le risque de progression en dysplasie ou en carcinome cervical grave.

2. Procédé selon la revendication 1, dans lequel l'étape (1) comprend en outre l'étape de détermination des rapports ARN/ADN des gènes E6 et/ou E7 de VPH.

3. Procédé selon la revendication 1 ou de la revendication 2, dans lequel l'activité de VPH mesurée est celle d'au moins un VPH de type hautement oncogène, par exemple les types hautement oncogène VPH16, VPH18, VPH31, VPH33 et VPH35.

4. Procédé selon l'une quelconque des revendication 1 à 3, dans lequel le transcript de E6 et/ou E7 comprend en outre des séquences nucléotidiques en aval du gène E7.

5. Procédé selon l'une quelconque des revendications précédentes, dans lequel les transcripts sont détectés par l'utilisation d'une ou plusieurs sondes, par exemple au moins une sonde de capture à queue qui est complémentaire d'un transcript d'au moins une portion de gène E6 ou E7 de VPH et qui comprend une queue polynucléotidique (par exemple polyd(A) polyd(T)) et une sonde de détection marquée (par exemple radionucléotidique, par exemple marquée au $^{32}$P ou au $^{125}$I) qui est complémentaire d'une portion différente du transcript du gène E6 ou E7 de VPH, comprenant en outre les étapes de : (a) mélange des sondes de capture et de détection avec un échantillon (par exemple un échantillon cervical) contenant des transcripts de VPH cibles pour former un complexe d'hybridation sonde de capture/transcript cible/sonde de détection,(b) mise en contact du complexe avec un support solide (par exemple des billes paramagnétiques, des billes de polystyrène et des bandelettes réactives de polystyrène ayant fixé sur elles un polynucléotide, par exemple poly d(T) et poly d(A), ce polynucléotide étant complémentaire de la queue polynucléotidique de la sonde de capture présent dans le complexe d'hybridation), dans des conditions permettant à l'hybridation de séquences nucléotidiques complémentaires de se produire, formant ainsi un complexe complexe d'hybridation/support solide, (c) séparation du complexe complexe d'hybridation/support solide du produit de l'étape (c) en ôtant le support solide ; et (d) détection du complexe complexe d'hybridation/support solide.

6. Procédé selon la revendication 5, dans lequel la sonde de capture est préhybridée avec le polynucléotide fixé au support solide.

7. Procédé selon la revendication 5 ou la revendication 6, dans lequel le VPH est choisi parmi les types hautement oncogènes VPH16, VPH18, VPH31, VPH33 et VPH35 et dans lequel les transcripts sont détectés par l'utilisation d'une ou plusieurs sondes essentiellement constituées d'une séquence nucléotidiques choisies parmi les séquences des sondes 16-1, 16-2, 16-3, 16-4, 16-5, 18-1, 18-2, 18-3, 18-4, 18-5, 18-6, 31-1, 31-2, 31-3, 31-4, 31-5, 33-1, 33-2, 33-3, 33-4, 33-5, 33-6, 35-1, 35-2, 35-3, 35-4, 35-5, ExHPV16E6*I, ExHPV16E6*II, ExHPV18E6*, ExHPV31E6*, ExHPV33E6* et ExHPV35E6*, comme décrit dans les figures 3 et 4.

8. Procédé selon l'une quelconque des revendications précédentes, comprenant en outre l'étape de détermination du mode de transcription des gènes E6 et/ou E7 (et/ou de transcripts épissés de ceux-ci) en utilisant un ensemble de sondes comprenant au moins des sondes, chacune étant complémentaire d'un transcript de VPH cible différent, et dans lequel l'ampleur de l'hybridation de chacune des sondes sur chacun des transcripts est quantifiée de façon à déterminer le mode de transcription.

9. Procédé selon l'une quelconque des revendications précédentes pour la détermination du risque de progression en dysplasie grave du col ou en carcinome cervical chez un individu femelle chez qui le VPH est présent dans le col, comprenant en outre l'étape de comparaison de l'activité mesurée de VPH avec l'activité associée avec (i) une dysplasie ou un carcinome in situ de gravité élevée ou (ii) le risque de progression du col en dysplasie grave ou en carcinome cervical, dans lequel la similitude déterminée d'après la comparaison donne une indication sur le risque de progression du col en dysplasie grave ou en carcinome cervical.

14

FIGURE 1

OBTAIN SAMPLE                                                    (1)
(e.g. cervical scrape)

SAMPLE TREATMENT                                                 (2)
(nucleic acids become available for hybridization)

ADD DETECTOR AND CAPTURE PROBES                        (3)

RETREIVE CAPTURE PROBES                        (4)
(and complexed target RNA and/or DNA molecules and detector probes)

SEPARATE HYBRIDIZATION COMPLEXES FROM MIXTURE  (5)

RECYCLE HYBRIDIZATION COMPLEXES                        (6)
(to other solid substrates)

QUANTITATE SIGNAL                                          (7)

15

Fig. 2

## Figure 3

| HPV | Probe No. | Oligonucleotide Sequence | | | | Target Nts. |
|-----|-----------|------|------|------|------|-------------|
| | | 5' | | | 3' | |
| 6 | | | | | | |
| | 6-1 | ACAACTGGTC | TATGGTCGTT | GCAGACGTGG | AGGCATTTGC | 111-150 |
| | 6-2 | TTGCAAAACA | CACAATTAAT | TTGCAACGTA | TGCATAGAT | 167-205 |
| | 6-3 | TTTAAAGGTT | GTGAATCTTG | TCCGTCCACT | TC | 644-675 |
| | 6-4 | GTTTCTGTAC | ACTGCACAAC | CAGTCGAACG | TTGCTG | 715-750 |
| | 6-5 | TATGTTTAGT | GTTCCCAACA | GAAGCTGTTG | CACTTC | 761-796 |
| 11 | 11-1 | CTCAACTGGT | CTATAGATGT | TGCAGACGTG | GAGGCATCTT | 112-151 |
| | 11-2 | CTGAATTTGC | AGAGTGTGCA | AAGAAAGATT | AAACGTCTTG | 152-191 |
| | 11-3 | TGTAACCCTA | CAGGGTCAGG | AGGCTGCAGG | TCTAGTACTA | 561-600 |
| | 11-4 | CCACAGCAAC | AGGTCAGTAT | TTGGTAATGT | TGTGTTAAAG | 669-708 |
| | 11-5 | CCGTCTGTGC | ACTCCACAAC | CAGTCGGACG | TTGCTG | 715-750 |
| | 11-6 | TATTTAGTGT | GCCCAGCAAA | AGGTCTTGTA | GTTGTCTGAT | 755-794 |
| 16 | | | | | | |
| | 16-1 | TCTGGGTCGC | TCCTGTGGGT | CCTGAAACAT | TGCAGTTC | 96-133 |
| | 16-2 | TCTAATATTA | TATCATGTAT | AGTTGTTTGC | AGCTCTGTGC | 150-189 |
| | 16-3 | TTGCAACAAA | AGGTTACAAT | ATTGTAATGG | GCTCTGTCCG | 701-740 |
| | 16-4 | GTCTACGTGT | GTGCTTTGTA | CGCACAACCG | AAGCGTAGAG | 747-786 |
| | 16-5 | TTCCTAGTGT | GCCCATTAAC | AGGTCTTCCA | AAGTACGAAT | 787-826 |
| 18 | | | | | | |
| | 18-1 | GTAGGGTCGC | CGTGTTGGAT | CCTCAAAGCG | CGCCATAG | 103-140 |
| | 18-2 | GTTATTTCTA | TGTCTTGCAG | TGAAGTGTTC | AGTTCCGTGC | 157-196 |
| | 18-3 | TAGAAGGTCA | ACCGGAATTT | CATTTTGGGG | CTCTAAATGC A | 627-667 |
| | 18-4 | TTACAACACA | TACACAACAT | TGTGTGACGT | TGTGGTTC | 752-789 |
| | 18-5 | GTCGTCTGCT | GAGCTTTCTA | CTACTAGCTC | AATTCTGGCT | 796-835 |
| | 18-6 | AGGACAGGGT | GTTCAGAAAC | AGCTGCTGGA | ATGCTCGAAG | 836-875 |
| 31 | | | | | | |
| | 31-1 | CAATTTCCGA | GGTCTTTCTG | CAGGATTTTT | GAACATGGCG | 103-142 |
| | 31-2 | GTTCATCGTA | GGGTATTTCC | AATGCCGAGC | TTAGTTCATG | 145-183 |
| | 31-3 | ACGAAGTGTA | GACTTACACT | GACAACAAAA | GGTAACG | 721-757 |
| | 31-4 | CTCTTGCAAT | ATGCGAATAT | CTACTTGTGT | GCTCTGTACA | 763-802 |
| | 31-5 | CAGTCTAGTA | GAACAGTTGG | GGCACACGAT | TCCAAATGA | 815-853 |

17

Figure 3 (Continued)

```
33-1    ATGCTTGGCA CAAATCATGC AATGTTCGTG GTTTTTCCTC           124-163
33-2    TTCCACGCAC TGTAGTTCAA TGTTGTGTAT AGTTGTCTCC A         164-204
33-3    GTATAGGTCA GTTGGTTCAG GATATAAATC TAAAACATAT           602-641
33-4    AGTGTGACAA CAGGTTACAA TGTAGTAATC AGCTGTGGCT           713-752
33-5    TTGCTGTACT GTTGACACAT AAACGAACTG TGGTGTT              756-792
33-6    TATTCACTGT GCCCATAAGT AGTTGCTGTA TGGTTCGTAG           798-837


35-1    TAAGGTCGTT CAGCTGGGTC CTGAAACATA CCGCACCTT            111-149
35-2    CATGGATGCT TTCTTCTACC TCGTTGCACA AATCATGCAG T         153-193
35-3    CACAGACGTA GTGTCGCCTC ACATTTACAA CAGGACGTT            740-778
35-4    ATCTTCCAAT TTACGTATGT CAATGTGTGT GCTCTGTACT           780-818
35-5    CTCTCTGTGA ACAGCCGGGG CACACTATTC CAAATGTGCT           829-867
```

## Figure 4

Consensus

---AAG<u>GT</u>AAGT---------------------PyPyPyPyPyPyNC<u>AG</u>GG---

      C      G                                                     T

exon →← ————— intron ————— →← —— exon

**Probe**                                                                 **Target**

ExHPV16E6*I    3' TTGTCAATGACGCTGCACTCCACATAATTGACAGTTTTCGG 5'
               5' AACAGTTACTGCGACGTGAGGTGTATTAACTGTCAAAAGCC 3'    HPV16E6*I
               207               226  409            429
               └——— exon ———┘ └——— exon———┘

ExHPV16E6*II  3' TTGTCAATGACGCTGCACTCTAGTAGTTCTTGTGCATCTC 5'
               5' AACAGTTACTGCGACGTGAGATCATCAAGAACACGTAGAG 3'    HPV16E6*II
               207               226  526            545
               └———exon———┘ └——— exon———┘

ExHPV18E6*    3' GTCATAACCTTGAATGTCTCCACGGACGCCACGGTCTTTG 5'
               5' CAGTATTGGAACTTACAGAGGTGCCTGCGGTGCCAGAAAC 3'    HPV18E6*
               209               228  406            430
               └———exon———┘ └——— exon———┘

ExHPV31E6*    3' GTCAATTGTCTTTGTCTCCACATATTGCACAGTTTC 5'
               5' CAGTTAACAGAAACAGAGGTGTATAACGTGTCAAAG 3'    HPV31E6*
               213            230  413           430
               └———exon———┘ └——— exon——┘

Figure 4 (Continued)

```
ExHPV33E6*    3' TTGGAAACGTTGCTAGACTCCACATAATATACAGTTTCTGG 5'
              5' AACCTTTGCAACGATCTGAGGTGTATTATATGTCAAAGACC 3'      HPV33E6*
                 212              231  414                434
                 L_____exon_____J  L_____exon_____J


ExHPV35E6*    3' TTCTTAATGTCGCCTCACTCCACATAATGTACAGTTTTTG 5'
              5' AAGAATTACAGCGGAGTGAGGTGTATTACATGTCAAAAAC 3'      HPV35E6*
                 224             243  426               445
                 L_____exon_____J  L_____exon_____J
```

16-4

16-3    16-5                          capture probes

0.51 Kb ribo

detector probe

do              ac              ac                              do

E6                                    E7              E1          mRNA target

200          400          600          800          1000

nucleotide number

Fig. 5

EP 0 502 994 B1

# Figure 6


```
PROBE 16-3  3'  GCCTGTCTCGGGTAATGTTATAACATTGGAAAACAACGTT  5'

    HPV 16  5'  CGGACAGAGCCCATTACAATATTGTAACCTTTTGTTGCAA  3'
            nt  701                                     740

    HPV 6       CtttaAaAcaaCATTtCcAaATaGTgACCTgTTGcTGtgg
    HPV 11      CtttaAcAcaaCATTACcAaATacTgACCTgTTGcTGtgg
    HPV 18      gaaccAcAacgtcacACAATgTTGTgtatgTgTtgTaagt
    HPV 31      CGGACAcAtCCaATTACAATATcGTtACCTTTTGTTGtcA
    HPV 33      CAGcCAcAGCtgATTACtAcATTGTAACCTgTTGTcaCAc
    HPV 35      CaGACAcctCCaATTAtAATATTGTAACgTccTGTTGtAA
```


```
Probe 16-4  3'  GAGATGCGAAGCCAACACGCATGTTTCGTGTGTGCATCTG  5'

    HPV 16  5'  CTCTACGCTTCGGTTGTGCGTACAAAGCACACACGTAGAC  3'
            nt  747                                     786

    HPV 6       CagcAacgTTCGacTGgttGTgCAgtGtACAgAaacAGAC
    HPV 11      CagcAacgTcCGacTGgttGTggAgtGCACAgACGgAGAC
    HPV 18      agCcAgaaTTgaGcTagtaGTAgAAAGCtCAgcaGacGAC
    HPV 31      gTCTACaCTTCGtTTGTGtGTACAgAGCACACAaGTAGAt
    HPV 33      caCcACagTTCGtTTaTGtGTcaAcAGtACAgcaagtGAc
    HPV 35      ggCgACaCTaCGtcTGTGtGTACAgAGCACACACATtGAC
```


```
Probe 16-5  3'  TAAGCATGAAACCTTCTGGACAATTACCCGTGTGATCCTT  5'

    HPV 16  5'  ATTCGTACTTTGGAAGACCTGTTAATGGGCACACTAGGAA  3'
            nt  787                                     826

    HPV 6       ATcaGagaagTGcAAcAgCTtcTgtTGGGaACACTAaacA
    HPV 11      ATcaGacaacTacAAGACCTttTgcTGGGCACACTAaatA
    HPV 18      cTTCGagcaTTccAgcAgCTGTTtcTGaacACcCTgtccT
    HPV 31      ATTCGcAtaTTGCAAGAgCTGTTAATGGGCtCAtTtGGAA
    HPV 33      cTacGaACcaTacAgcAaCTacTtATGGGCACAgTgaatA
    HPV 35      ATaCGTAaaTTGGAAGAttTaTTAATGGGCACAtTtGGAA
```

22

*Fig. 7*

FIGURE 8A

Positive Target

FIGURE 8B